(19) **Europäisches Patentamt / European Patent Office / Office européen des brevets**

(11) **EP 4 511 646 B1**

(12) ## EUROPEAN PATENT SPECIFICATION

(45) Date of publication and mention of the grant of the patent:
**22.10.2025 Bulletin 2025/43**

(21) Application number: **23720614.9**

(22) Date of filing: **20.04.2023**

(51) International Patent Classification (IPC):
**G01N 27/12** (2006.01)

(52) Cooperative Patent Classification (CPC):
**G01N 27/127**

(86) International application number:
**PCT/EP2023/060303**

(87) International publication number:
**WO 2023/203141 (26.10.2023 Gazette 2023/43)**

(54) **ZNO NANORODS PROVIDED WITH ORTHOGONAL OXIDIZED COPPER NANOPLATES AND CORRESPONDING GAS SENSOR**

ZNO-NANOSTÄBCHEN MIT ORTHOGONALEN OXIDIERTEN KUPFERNANOPLATTEN UND ENTSPRECHENDER GASSENSOR

NANOTIGES DE ZNO POURVUES DE NANOPLAQUES DE CUIVRE OXYDÉES ORTHOGONALES ET CAPTEUR DE GAZ CORRESPONDANT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.04.2022 LU 501895**

(43) Date of publication of application:
**26.02.2025 Bulletin 2025/09**

(73) Proprietor: **LUXEMBOURG INSTITUTE OF SCIENCE AND TECHNOLOGY (LIST)**
**4362 Esch-sur-Alzette (LU)**

(72) Inventors:
• **LETURCQ, Renaud**
  **2518 Luxembourg (LU)**
• **THOMANN, Jean-Sébastien**
  **54730 Gorcy (FR)**
• **BHUSARI, Rutuja**
  **4035 Esch-sur-Alzette (LU)**

(74) Representative: **Ipsilon Benelux**
  **76, rue de Merl**
  **2146 Luxembourg (LU)**

(56) References cited:
• RAHMATI ALI ET AL: "Heteroepitaxial ZnO/CuO thin film and nanorods array: photoconductivity and field emission effect", JOURNAL OF MATERIALS SCIENCE: MATERIALS IN ELECTRONICS, CHAPMAN AND HALL, LONDON, GB, vol. 28, no. 17, 26 May 2017 (2017-05-26), pages 13032 - 13040, XP036300838, ISSN: 0957-4522, [retrieved on 20170526], DOI: 10.1007/S10854-017-7135-8
• LU PING ET AL: "CuO nanosheets/ZnO nanorods synthesized by a template-free hydrothermal approach and their optical and magnetic characteristics", CERAMICS INTERNATIONAL, ELSEVIER, AMSTERDAM, NL, vol. 43, no. 13, 28 April 2017 (2017-04-28), pages 9798 - 9805, XP085066542, ISSN: 0272-8842, DOI: 10.1016/J.CERAMINT.2017.04.159
• SIMON QUENTIN ET AL: "CuO/ZnO Nanocomposite Gas Sensors Developed by a Plasma-Assisted Route", CHEMPHYSCHEM, vol. 13, no. 9, 18 June 2012 (2012-06-18), DE, pages 2342 - 2348, XP055981454, ISSN: 1439-4235, DOI: 10.1002/cphc.201101062

EP 4 511 646 B1

- **SETT AVIK ET AL: "Selective detection of multiple VOCs employing zinc oxide nanorods and principle component", 2020 4TH INTERNATIONAL CONFERENCE ON ELECTRONICS, MATERIALS ENGINEERING & NANO-TECHNOLOGY (IEMENTECH), IEEE, 2 October 2020 (2020-10-02), pages 1 - 6, XP033867824, DOI: 10.1109/IEMENTECH51367.2020.9270117**

**Description**

**Technical field**

The invention is directed to the field of metal oxide heterostructures for various applications including gas sensing, photocatalysis and water splitting.

**Background art**

Prior art patent document published CN 106814113 A discloses a semiconductor oxide gas sensor based on ZnO nanorods onto which CuO particles are grown, so as to provide a good sensitivity to $H_2S$. The CuO particles are uniformly distributed on the ZnO nanorods and show a diameter comprised between 20 and 50nm. The resulting gas sensor shows a good sensitivity to $H_2S$ and also a good selectivity compared with other gases like $SO_2$, $Cl_2$ and $O_2$, in particular at 50°C.

Prior art patent document published CN 107537501 A discloses a method for producing ZnO/-CuO composite material intended to be used for gas sensing, photocatalysis and water splitting. The ZnO/-CuO composite material comprises a flower-like hierarchical structure of ZnO matrix and CuO nanoparticles attached to the surface of said matrix. The size of the CuO nanoparticles is comprised between 10and 50nm whereas the size of the hierarchical structure of ZnO is comprised between 10 and 20$\mu$m.

Rutuja Bhusari, Jean-Sébastien Thomann, Jérôme Guillot, Renaud Leturcq, "Morphology control of copper hydroxide based nanostructures in liquid phase synthesis", Journal of Crystal Growth, Volume 570, 2021, 126225, ISSN 0022-0248, https://doi.org/10.1016/j.icrysgro.2021.126225 (https://www.sciencedirect.com/science/article/pii/S0022024821002001) discloses synthesis and study of copper hydroxide based nanostructures as templates for formation of CuO, more particularly the template-free bottom-up synthesis and shape control of copper hydroxide based nanostructures grown in liquid phase, from 1D nanowires to 2D layered nanoplatelets and 3D nanocrystals.

RAHMATI ALI ET AL, "Heteroepitaxial ZnO/-CuO thin film and nanorods array: photoconductivity and field emission effect", JOURNAL OF MATERIALS SCIENCE: MATERIALS IN ELECTRONICS, CHAPMAN AND HALL, LONDON, GB, vol. 28, no. 17, doi:10.1007/S10854-017-7135-8, ISSN 0957-4522, (20170526), pages 13032 - 13040, (20170526), discloses a metal oxide material according to the preamble of claim 1.

LU PING ET AL, "CuO nanosheets/ZnO nanorods synthesized by a template-free hydrothermal approach and their optical and magnetic characteristics", CERAMICS INTERNATIONAL, ELSEVIER, AMSTERDAM, NL, (20170428), vol. 43, no. 13, doi:10.1016/J.CERAMINT.2017.04.159, ISSN 0272-8842, pages 9798 - 9805, discloses a metal oxide material according to the preamble of claim 1.

SIMON QUENTIN ET AL, "CuO/ZnO Nanocomposite Gas Sensors Developed by a Plasma-Assisted Route", CHEMPHYSCHEM, DE, (20120618), vol. 13, no. 9, doi:10.1002/cphc.201101062, ISSN 1439-4235, pages 2342 - 2348, discloses a gas sensor with a gas sensing layer comprising ZnO nanorods and CuO nanoplates.

The gas sensitivity of the above described ZnO/CuO composite materials, remains limited, in particular at room temperature or controlled temperatures below 200°C. It is therefore desirable to increase the gas sensitivity and/or selectivity of these materials.

**Summary of invention**

Technical Problem

The invention has for technical problem to overcome at least one drawback of the above cited prior art. More specifically, the invention has for technical problem to provide a metal oxide heterostructure with an increased chemiresistive gas sensitivity and/or selectivity.

Technical solution

The invention is directed to a metal oxide material comprising ZnO nanorods; and oxidized copper particles attached to the ZnO nanorods; wherein the oxidized copper particles are nanoplates extending transversally to the ZnO nanorods, said transversal oxidized copper nanoplates have a normal parallel to a longitudinal axis of the ZnO nanorods or inclined relative to said longitudinal axis by not more than 45°; and the ZnO nanorods provided with transversal nanoplates of oxidized copper have a number of the transversal nanoplates of oxidized copper that is comprised between 10 and 100 per $\mu$m of length of ZnO nanorod.

According to a preferred embodiment, the ZnO nanorods have a length comprised between 1 and 10$\mu$m.

According to a preferred embodiment, the ZnO nanorods have a diameter comprised between 50 and 600nm.

According to a preferred embodiment, the ZnO nanorods extend along a longitudinal axis and the transversal oxidized copper nanoplates are perpendicular to the longitudinal axis within a tolerance of $\pm$15°, preferably within a tolerance of $\pm$10°.

According to a preferred embodiment, the ZnO nanorods provided with transversal nanoplates of oxidized copper are obtainable by the method of the invention.

The invention is also directed to a gas sensor comprising a substrate; at least two electrodes deposited on the substrate; a gas sensing layer deposited on the at least two electrodes and on the substrate between said at least two electrodes, said gas sensing layer comprising the metal oxide material of the invention and showing an electrical resistivity that varies when contacted by the gas being at least one of $O_2$, $H_2$, CO, ethanol and $NO_2$.

Advantageously, the oxidized copper forming the transversal nanoplates comprises CuO. It can also comprise $Cu_2(OH)_3Cl$ and $Cu(OH)_2$.

Advantageously, the gas sensor further comprises an electrical resistor dimensioned for bringing the substrate, the at least two electrodes and the gas sensing layer at a temperature of at least 100°C and up to 250°C.

The invention is also directed to a method of synthetizing ZnO nanorods provided with Cu-based nanoplates, comprising the following successive steps: (a) preparing ZnO nanorods; (b) mixing the ZnO nanorods with a Cu precursor in a solution; (c) increasing the pH of the solution, so as to form $Cu(OH)_2$ nanowires in the solution; (d) lowering the pH of the solution, so as to form $Cu_2(OH)_3Cl$ nanoplates on the ZnO nanorods.

Advantageously, the solution is water based.

According to a preferred embodiment, the method comprises the further step: (e) annealing the ZnO nanorods provided with the $Cu_2(OH)_3Cl$ nanoplates so as to transform at least partially the $Cu_2(OH)_3Cl$ nanoplates into CuO nanoplates.

According to a preferred embodiment, annealing at step (e) is achieved at a temperature of at least 100°C during at least one hour, preferably at least two hours.

According to a preferred embodiment, annealing at step (e) is achieved at a temperature of at least 200°C during at least one hour, preferably at least two hours.

According to a preferred embodiment, step (c) comprises adding NaOH so as to increase the pH to at least 12.

According to a preferred embodiment, step (d) comprises adding HCl so as to lower the pH to a value comprised between 6 and 8.

Advantageously, the Cu precursor comprises $CuCl_2$.

Advantages of the invention

The invention is particularly interesting in that it provides a metal oxide material and a corresponding gas sensor showing an improved chemiresistive sensitivity in the presence of different gases, essentially by increasing the total contact surface with the gas per unit of metal oxide material.

The range of applications of the metal oxide material of the invention is broad and therefore is not limited to gas sensing and, in the context of gas sensing, is not limited to sensing a unique or specific type of gas. the working principle of chemiresistive gas sensor is the change in resistance due to chemical redox reactions and is based on the Ohm's law, $V = R \cdot I$. When a target gas interacts with the oxygen adsorbed or with the elements on the surface of a chemiresistive sensor, there occurs a reaction. This leads to change in the resistance of the material. The ability of the metal oxide surface to react with the target gas depends on the properties of the surface and the oxygen species adsorbed on the surface. The adsorption of oxygen at the surface changes the resistance of the semiconductor material formed by the metal oxide.

**Brief description of the drawings**

Figure 1 illustrates the heterostructure of ZnO nanorods with transversal nanoplates of CuO, according to the invention.

Figure 2 illustrates the synthesis of the ZnO nanorods with transversal nanoplates of $Cu_2(OH)_3Cl$, according to the invention.

Figure 3 illustrates the geometry of nanoparticles versus nanoplates.

Figure 4 illustrates a gas sensor according to two variants, according to the invention.

Figure 5 is a graphic illustrating the electrical behavior of a gas sensor according to the invention in contact with various gases.

**Description of an embodiment**

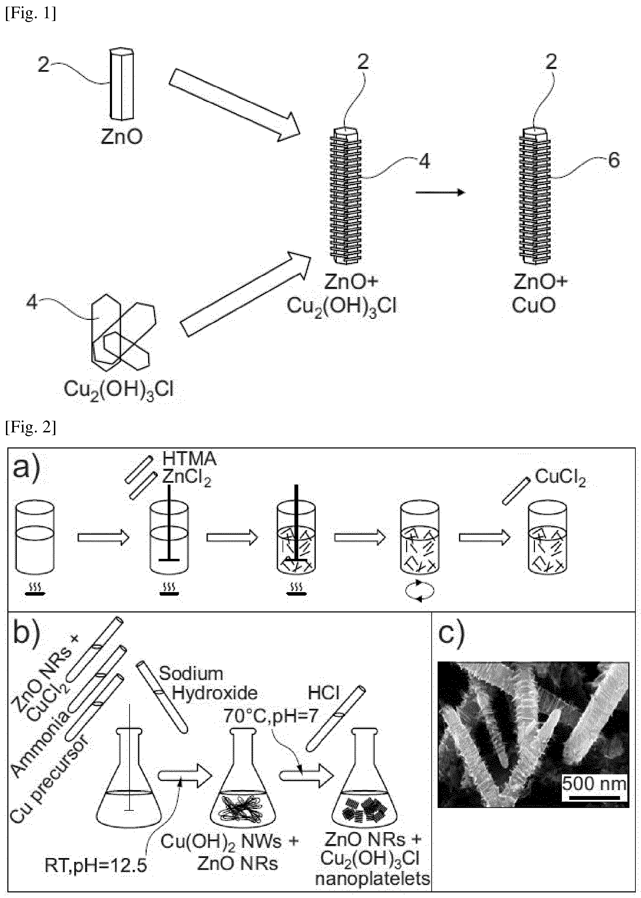

Figure 1 illustrates the heterostructure of ZnO nanorods provided with transversal nanoplates of CuO, according to the invention.

As this is apparent, ZnO nanorods 2 are provided and $Cu_2(OH)_3Cl$ nanoplates 4 that are growth on the ZnO nanorods 2. The ZnO nanorods 2 with the $Cu_2(OH)_3Cl$ nanoplates 4 are then annealed resulting in a conversion of the $Cu_2(OH)_3Cl$ nanoplates 4 into CuO nanoplates 6.

As this is apparent, the nanoplates of $Cu_2(OH)_3Cl$ nanoplates 4 and more particularly the CuO nanoplates 6 are perpendicular to the longitudinal axis of the ZnO nanorod 2 and are distributed evenly along and around the nanorod 2. This particular arrangement of CuO nanoplates on ZnO nanorods is particularly advantageous in that it maximizes the effective surface of the copper oxide nanoplates per nanorod and thereby increases the catalytic effect useful for the various applications including gas sensing, photocatalysis and water splitting.

Figure 2 illustrates a method for synthetizing the above ZnO nanorods 2 with the $Cu_2(OH)_3Cl$ nanoplates 4.

More specifically, section a) of figure 2 illustrate the synthetizing of ZnO nanorods, comprising essentially mixing $ZnCl_2$ with Hexamethylenetetramine (HTMA), also known as methenamine, hexamine, or urotropin, and being heterocyclic organic compound with the formula $(CH_2)_6N_4$, with water, heating and mixing, and centrifugating. More specifically, the method comprises the following steps:

- as illustrated in section a)-1 of figure 2, providing a solution, e.g. water, and heating said solution, e.g. to a temperature comprised between 50 and 100°C, preferably comprised between 60 and 80°C;

- as illustrated in section a)-2 of figure 2, adding and dissolving $ZnCl_2$ and Hexamethylenetetramine (HTMA) to the solution, while keeping heating the solution, preferably with equimolar amounts;

- as illustrated in section a)-3 of figure 2, mixing the above solution while heating said solution preferably at a slightly higher temperature comprised between 70 and 100°C, and this during a certain duration, e.g. of at least 60 minutes;

- as illustrated in section a)-4 of figure 2, centrifugating the solution, while having stopped heating, e.g. at room temperature, so as to separate ZnO nanorods from the solution.

As illustrated in section a)-5 of figure 2, the centrifugated ZnO nanorods can be added to a solvent solution, like ethanol, optionally with $CuCl_2$, in preparation the further steps for growing the copper oxides on the ZnO nanorods.

The above method of synthetizing ZnO nanorods is as such known, notably from the patent application WO 2019/057786 A1 assigned to the applicant of the present application. Other methods can also be considered.

Section b) of figure 2 illustrates the growing of $Cu_2(OH)_3Cl$ nanoplates on the above synthetized ZnO nanorods. It is to be understood that the ZnO nanorods do not necessarily need to be synthetized according to the above method as illustrated in section a) of figure 2.

As this is apparent in section b) of figure 2, a solution of ZnO nanorods and copper precursor such as $CuCl_2$ in a solution, being for instance water, is mixed with ammonia $NH_4OH$ and sodium hydroxide NaOH so as to increase the pH of the resulting solution, e.g up to at least 12, preferably at room temperature. This results in obtaining a precipitate with $Cu(OH)_2$ nanowires and the ZnO nanorods.

Thereafter, the solution is heated, preferably between 60 and 80 °C, and the pH of the solution is lowered by adding an acid, like chloride acid HCl, to a value of not more than 8 and more than 5, preferably comprised between 6 and 7.5, where botallackite ($Cu_2(OH)_3Cl$) nanoplates are formed. Reducing the pH to a lower value would form clinoatacamite ($Cu_2(OH)_3Cl$) nanocrystals which is not desired.

The obtained ZnO nanorods with $Cu_2(OH)_3Cl$ nanoplates are illustrated in section c) of figure 2. The solution can then be centrifugated and washed so as to separate the ZnO nanorods with $Cu_2(OH)_3Cl$ nanoplates for being then applied onto a substrate for forming a gas sensor, a photocatalyst and a water splitter. In alternative, the ZnO nanorods with $Cu_2(OH)_3Cl$ nanoplates can be stored in a solvent, like ethanol.

As stated here above in connection with figure 1, the ZnO nanorods with $Cu_2(OH)_3Cl$ nanoplates are annealed for converting the $Cu_2(OH)_3Cl$ nanoplates into CuO nanoplates. The annealing is done at a temperature comprised between 100 and 400°C with a duration of at least one hour, preferably at least two hours.

The conversion of the $Cu_2(OH)_3Cl$ nanoplates into CuO nanoplates has been confirmed by high resolution X-ray photoelectron spectroscopy (XPS) of samples, the Cu $2p_{3/2}$ peak showing a substantially higher peak in the atom counts for the binding energy of 933.6 eV corresponding to CuO after annealing compared with before annealing. $Cu_2(OH)_3Cl$ or $Cu(OH)_2$ are also present as confirmed by a peak for the binding energy of 934.8 eV, with a peak hight reduced after annealing as compared with before annealing. As an examples, the mass ratio between Cu in CuO and Cu in copper hydroxide ($Cu_2(OH)_3Cl$ or $Cu(OH)_2$) is evaluated by a fit of the XPS Cu $2p_{3/2}$ peak, and is found to be 0.5 before annealing, and 2.5 after 2 hours annealing at 400 °C.

The conversion of the $Cu_2(OH)_3Cl$ nanoplates into CuO nanoplates has also been confirmed by X-ray diffraction analysis (XRD) of samples, where the peaks at angles of 15.5 degrees, corresponding to botallackite $Cu_2(OH)_3Cl$, at angles of 16.7 degrees, corresponding to paratacamite $Cu_2(OH)_3Cl$, and at angles of 16.2 and 23.8 degrees, corresponding to $Cu(OH)_2$, are suppressed after annealing, and the peaks at angles of 35.5 and 38.9 degrees, corresponding to CuO, appear after annealing.

The conversion of the $Cu_2(OH)_3Cl$ nanoplates into CuO nanoplates also changes the optical properties of the composite material. For instance, optical absorbance analysis of samples in the wavelength range from 250 nm to 1500 nm have been made and revealed that the annealed composite material showed a substantially higher absorbance in the visible range (from 300 to 800 nm) and also that the absorbance is higher for samples annealed at higher temperatures compared with those annealed at lower temperatures, these temperatures being comprised between 100 and 400°C.

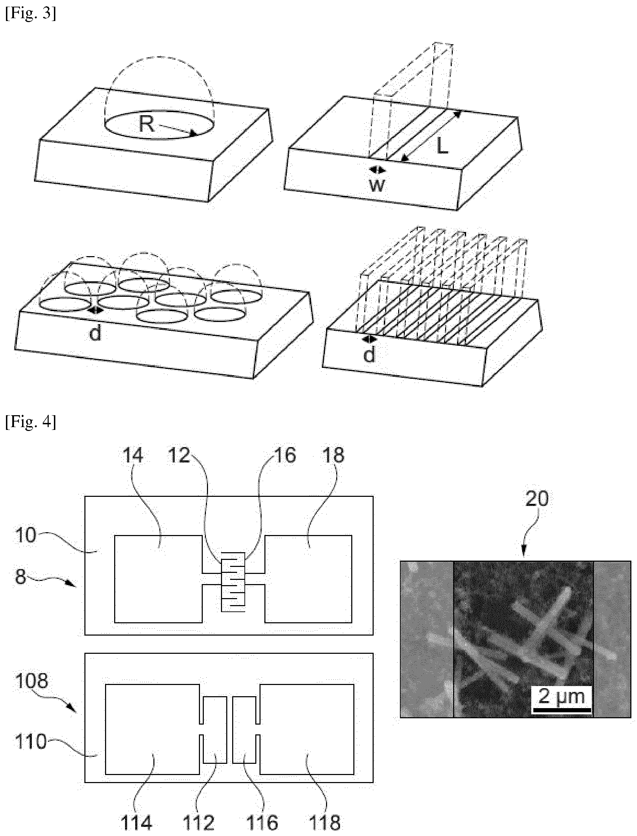

Figure 3 illustrates the geometry of nanoparticles versus nanoplates.

The top left image shows a droplet or particle-shaped element attached to a support, the lower left image shows an array arrangement of the droplet or particle shaped elements. The top right image shows a plate-shaped element attached to a support and the lower right image shows an evenly distributed arrangement of the plate-shaped elements on the support.

For the droplet or particle-shaped element, we have

$$\frac{P}{A} = \frac{2}{R}$$

$$\lim_{R \to 0} \frac{P}{A} = \infty$$

$$\lim_{R \to 0} P = 0$$

where $R$ is the radius of the droplet or particle-shaped element, P and A are the perimeter and surface on the support, respectively, of the droplet or particle-shaped element. It follows that droplet or particle-shaped element of a nano radius will show a surface that proportionally diminishes compared with the perimeter, i.e. the place that it occupies on the substrate.

If we consider the above-mentioned array of droplet or particle-shaped elements, we have

$$\frac{P_{tot}}{A_{tot}} = \frac{2\pi R}{\frac{\sqrt{3}}{4}(2R + d)^2}$$

$$\lim_{R \to 0} \frac{P_{tot}}{A_{tot}} = 0$$

where R is the radius of the droplet or particle-shaped element, $P_{tot}$ and $A_{tot}$ are the total perimeter and total covered surface of the droplet or particle-shaped elements, and d is the distance between directly adjacent droplet or particle-shaped elements. It follows that an array of droplet or particle-shaped elements will show a total perimeter, i.e. a sum of the perimeters of all droplet or particle-shaped element of the array, being representative of the active surface that can be contacted by a gas, that will progressively become smaller than the total surface that the droplet or particle-shaped elements occupy on the substrate, as the radius R of the droplet or particle-shaped elements reaches a nanoscale.

For the plate-shaped element, we have

$$\frac{P}{A} = \frac{2}{w} + \frac{2}{L}$$

$$\lim_{w \to 0} \frac{P}{A} = \infty$$

$$P = 2L + 2w$$

$$\lim_{w \to 0} P = 2L$$

where L is the length and w the width (in fact thickness) of the plate-shaped element, P and A are the perimeter and surface on the support, respectively, of the plate-shaped element. It follows that plate-shaped element of a nano width w will keep a perimeter proportional to its length L while the place that it occupies on the substrate diminishes.

If we consider the above-mentioned even distribution of plate-shaped elements, we have

$$\frac{P_{tot}}{A_{tot}} = \frac{2L + 2w}{(w + d)L}$$

$$\lim_{w \to 0} \frac{P_{tot}}{A_{tot}} = \frac{2}{d}$$

where $P_{tot}$ and $A_{tot}$ are the total perimeter and total covered surface of the plate-shaped elements, and d is the distance between directly adjacent plate-shaped elements. It follows that reducing the width w to a nano scale maintains a fixed proportionality between the total perimeter, being representative of the of the active surface that can be contacted by a gas, with the total surface on the support that is occupied by the plate-shaped elements.

The above demonstrates the advantages of the above transversal arrangement of copper oxide nanoplates on the ZnO nanorods, with regard to optimizing the total contact surface for ambient gas for a given number and size of the nanorods. This thereby optimizes the sensitivity of the composite material.

The above-described ZnO nanorods show a length that can be comprised between 1 and 10$\mu$m and a diameter comprised between 50 and 600 nm, resulting in a shape factor comprised between and 1.7 and 200. The above-described transversal nanoplates of copper oxide are distributed along the length of the ZnO nanorods with a density comprised between 10 and 100 per $\mu$m. The transversal nanoplates of copper oxide can show a thickness (corresponding to w in figure 3) comprised between 15 and 35nm. The length (corresponding to L in figure 3) can be comprised between 800 and 2300nm, and the height (perpendicular to w and L in figure 3) can be comprised between 300 and 630nm. The above ranges are also disclosed, each, in isolation and each range is disclosed with its lower limit only and also with its upper limit only.

Also, the above-described transversal nanoplates of copper oxide are transversal to the ZnO nanorods, a normal parallel to a longitudinal axis of the ZnO nanorods or inclined relative to said longitudinal axis by not more than 45°, more preferably perpendicular to the ZnO nanorods within a tolerance of $\pm15°$, even more preferably within a tolerance of $\pm10°$.

Also, the above-described ZnO nanorods show a polygonal cross-section forming facets onto which the transversal nanoplates of copper oxide are attached.

Figure 4 illustrates two embodiments of a gas sensor according to the invention.

The first embodiment is a gas sensor 8 comprising a substrate 10, a first electrode 12 and a first contact area electrically connected to the first electrode 12. Similarly, the gas sensor comprises a second electrode 16 and a second contact area electrically connected to the second electrode 12. As this is apparent the first and second electrodes 12 and 16 are interdigitated. The first and second electrodes 12 and 16, and the first and second contact areas 14 and 18 are formed by deposition of an electrically conductive material, e.g. copper based, on the substrate 10. The first and second electrodes 12 and 16 as well as directly adjacent areas of the substrate 10 are covered by a layer of ZnO nanorods with transversal nanoplates of CuO as detailed here above, forming a gas sensing layer 20.

The second embodiment is a gas sensor 108 similar to the gas sensor 8 of the first embodiment, comprising also first and second contact areas 114 and 118 but where, however, the first and second electrodes 112 and 116 are not interdigitated. Similarly to the first embodiment, the first and second electrodes 112 and 116 as well as directly adjacent areas of the substrate 110 are covered by the gas sensing layer 20.

The gas sensors 8 and 108 can further comprise an electrical resistor dimension for bringing the substrate, the at least two electrodes and the gas sensing layer at a temperature of at least 100°C and up to 250°C.

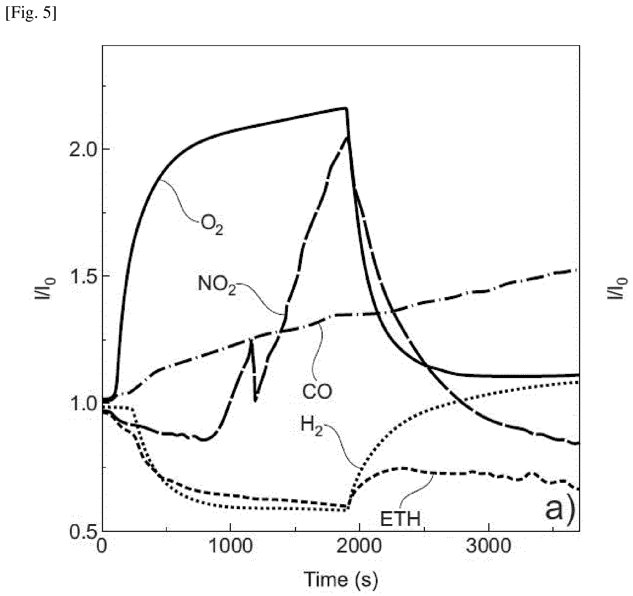

Figure 5 is a graphic illustrating the electrical behavior of the gas sensor according to the invention in contact with various gases.

The above-described gas sensor has been tested in contact with various gases and its electrical behavior has been observed and reported in the graphic in figure 5. An electrical source, e.g. a voltage source, has been electrically connected to the contact areas of the gas sensor, while contacting its gas sensing layer with $N_2$ and maintaining the gas sensor at a controlled temperature of 150°C, resulting in a stabilized nominal current $I_0$ flowing through the gas sensor. The gas sensor has then been brought into contact different types of gas as will be explained here after, and the current $I$ has been measured. To that end, the ambient atmosphere of the gas sensor is saturated successively with each of the different types at given concentrations.

In practice, the gas sensor is located in a generally closed chamber that is fed with either pure $N_2$ and thereafter with the gas to be tested, in the case the gas to be tested is $O_2$, or dry air and thereafter with the gas to be tested, in the case the gas to be tested is $H_2$, CO, $NO_2$ or ethanol. The chamber comprises at least one exhaust so as to allow the gas to be tested to flow, i.e. to be renewed in the chamber, e.g. about every 30 seconds.

The gas sensor has been brought into contact with $O_2$ at a concentration of 20.9% mixed $N_2$ as a carrier gas, during 1800 seconds (i.e. 30 minutes). We can observe a rapid increase in the current response, where $I/I_0$ reaches 2 after about 600-700 seconds. Thereafter the increase in the current response is less steep. After applying the above gas $O_2$ containing gas, for instance during 1800 seconds, pure $N_2$ is applied as before applying said $O_2$ containing gas. We can see a rapid decrease in the current response.

The gas sensor has also been brought into contact with $H_2$ at 2% in $N_2$, also during 1800 seconds, similarly to the above $O_2$ and $N_2$ gas mixture. We can observe a current response that is opposed to the one with $O_2$, i.e. where the current diminishes instead of increasing. The current response $I/I_0$ reaches 0.6 after about 750 seconds, which is comparable with the above current response with $O_2$.

The gas sensor has also been brought into contact with CO at 20ppm in air, also during 1800 seconds, similarly to the above $O_2$-$N_2$ and $H_2$-$N_2$ gas mixtures. The response observed for CO is much different than that observed for $H_2$, even though both are reducing gasses.

The gas sensor has also been brought into contact with $NO_2$ at 8.26 ppm in air, also during 1800 seconds, similarly to the above gas mixtures. We can observe a substantial increase of the current response. The delayed response observed with this gas is due to an experimental artifact due to the large dilution of this specific gas. After stopping application of the above gas mixture, a rapid decrease of the current response can be observed.

The gas sensor has also been brought into contact with ethanol (ETH) at 100 ppm in air, also during 1800 seconds, similarly to the above gas mixtures. We can observe a current response that is similar to the one of $H_2$ in $N_2$, i.e. a decrease in the current response, whereas after application of the above test gas mixture, the recovery current behavior is quite different from the one with $H_2$ in $N_2$.

In summary, we observe in figure 5 a good sensitivity and selectivity to the tested gases, thereby showing the suitability and ability of the composite material of the invention and corresponding sensor for gas sensing applications, in particular for the above tested gases.

**Claims**

1. A metal oxide material comprising:

   ZnO nanorods (2); and
   oxidized copper particles attached to the ZnO nanorods (2), the oxidized copper particles (6) being nanoplates extending transversally to the ZnO nanorods;
   **characterized in that**
   said transversal oxidized copper nanoplates have a normal parallel to a longitudinal axis of the ZnO nanorods or inclined relative to said longitudinal axis by not more than 45°; and
   the ZnO nanorods (2) provided with transversal nanoplates of oxidized copper (4; 6) have a number of the transversal nanoplates of oxidized copper that is comprised between 10 and 100 per $\mu$m of length of ZnO nanorod.

2. The metal oxide material of claim 1, wherein the ZnO nanorods (2) have a length comprised between 1 and 10$\mu$m.

3. The metal oxide material of one of claims 1 and 2, wherein the ZnO nanorods (2) have a diameter comprised between 50 and 600nm.

4. The metal oxide material of any one of claims 1 to 3, wherein the transversal oxidized copper nanoplates (6) are perpendicular to the longitudinal axis of the ZnO nanorods within a tolerance of $\pm15°$, preferably within a tolerance of $\pm10°$.

5. The metal oxide material of any one of claims 1 to 4, wherein the ZnO nanorods (2) provided with transversal nanoplates of oxidized copper (4; 6) are obtainable by the method of any one of claims 7-12.

6. A gas sensor (8; 108) comprising:

a substrate (10; 110);
at least two electrodes (12, 16; 112, 116) deposited on the substrate (10; 110);
a gas sensing layer (20) deposited on the at least two electrodes (12, 16; 112, 116) and on the substrate (10; 110) between said at least two electrodes, said gas sensing layer (20) comprising the metal oxide material of any one of claims 1 to 5 and showing an electrical resistivity that varies when contacted by the gas being at least one of $O_2$, $H_2$, CO, ethanol and $NO_2$.

7. A method of synthetizing ZnO nanorods provided with Cu-based nanoplates, comprising the following successive steps:

(a) preparing ZnO nanorods;
(b) mixing the ZnO nanorods with a Cu precursor in a solution;
(c) increasing the pH of the solution, so as to form $Cu(OH)_2$ nanowires in the solution;
(d) lowering the pH of the solution, so as to form $Cu_2(OH)_3Cl$ nanoplates (4) on the ZnO nanorods (2).

8. The method of claim 7, comprising the further step:
(e) annealing the ZnO nanorods (2) provided with the $Cu_2(OH)_3Cl$ nanoplates (4) so as to transform at least partially the $Cu_2(OH)_3Cl$ nanoplates (4) into CuO nanoplates (6).

9. The method of claim 8, wherein annealing at step (e) is achieved at a temperature of at least 100°C during at least one hour, preferably at least two hours.

10. The method of claim 8, wherein annealing at step (e) is achieved at a temperature of at least 200°C during at least one hour, preferably at least two hours.

11. The method of any one of claims 7 to 10, wherein step (c) comprises adding NaOH so as to increase the pH to at least 12.

12. The method of any one of claims 7 to 11, wherein step (d) comprises adding HCl so as to lower the pH to a value comprised between 6 and 8.

**Patentansprüche**

1. Ein Metalloxidmaterial, umfassend:

ZnO-Nanoröhren (2); und
oxidierte Kupferpartikel, die an den ZnO-Nanoröhren (2) befestigt sind, wobei die oxidierten Kupferpartikel (6) Nanoplättchen sind, die transversal zu den ZnO-Nanoröhren verlaufen;
**dadurch gekennzeichnet, dass**
die transversalen oxidierten Kupfer-Nanoplättchen eine Normale parallel zu einer Längsachse der ZnO-Nanoröhren oder relativ zu dieser Längsachse um nicht mehr als 45° geneigt haben; und
dass die ZnO-Nanoröhren (2), die mit transversalen Nanoplättchen aus oxidiertem Kupfer (4; 6) versehen sind, eine Anzahl der transversalen Nanoplättchen aus oxidiertem Kupfer aufweisen, die zwischen 10 und 100 pro um Länge der ZnO-Nanoröhre liegt.

2. Das Metalloxidmaterial nach Anspruch 1, wobei die ZnO-Nanoröhren (2) eine Länge zwischen 1 und 10 um aufweisen.

3. Das Metalloxidmaterial nach einem der Ansprüche 1 und 2, wobei die ZnO-Nanoröhren (2) einen Durchmesser zwischen 50 und 600 nm aufweisen.

4. Das Metalloxidmaterial nach einem der Ansprüche 1 bis 3, wobei die transversalen oxidierten Kupfer-Nanoplättchen (6) innerhalb einer Toleranz von $\pm15°$, vorzugsweise innerhalb einer Toleranz von $\pm10°$, senkrecht zur Längsachse der ZnO-Nanoröhren verlaufen.

5. Das Metalloxidmaterial nach einem der Ansprüche 1 bis 4, wobei die ZnO-Nanoröhren (2), die mit transversalen Nanoplättchen aus oxidiertem Kupfer (4; 6) versehen sind, durch das Verfahren nach einem der Ansprüche 7-12 erhältlich sind.

**6.** Ein Gassensor (8; 108), umfassend: ein Substrat (10; 110); mindestens zwei Elektroden (12, 16; 112, 116), die auf dem Substrat (10; 110) aufgebracht sind; eine Gasschicht (20), die auf den mindestens zwei Elektroden (12, 16; 112, 116) und auf dem Substrat (10; 110) zwischen den mindestens zwei Elektroden aufgebracht ist, wobei die Gasschicht (20) das Metalloxidmaterial nach einem der Ansprüche 1 bis 5 umfasst und eine elektrische Resistivität aufweist, die sich bei Kontakt mit dem Gas, das mindestens eines von $O_2$, $H_2$, CO, Ethanol und $NO_2$ ist, verändert.

**7.** Ein Verfahren zur Synthese von ZnO-Nanoröhren, die mit Cu-basierten Nanoplättchen versehen sind, umfassend die folgenden aufeinanderfolgenden Schritte:

(a) Herstellung von ZnO-Nanoröhren;
(b) Mischen der ZnO-Nanoröhren mit einem Cu-Vorläufer in einer Lösung;
(c) Erhöhung des pH-Werts der Lösung, um $Cu(OH)_2$-Nanodrähte in der Lösung zu bilden;
(d) Senkung des pH-Werts der Lösung, um $Cu_2(OH)_3Cl$-Nanoplättchen (4) auf den ZnO-Nanoröhren (2) zu bilden.

**8.** Das Verfahren nach Anspruch 7, umfassend den weiteren Schritt:
(e) Glühen der ZnO-Nanoröhren (2), die mit den $Cu_2(OH)_3Cl$-Nanoplättchen (4) versehen sind, um die $Cu_2(OH)_3Cl$-Nanoplättchen (4) zumindest teilweise in CuO-Nanoplättchen (6) umzuwandeln.

**9.** Das Verfahren nach Anspruch 8, wobei das Glühen in Schritt (e) bei einer Temperatur von mindestens 100°C für mindestens eine Stunde, vorzugsweise mindestens zwei Stunden, erfolgt.

**10.** Das Verfahren nach Anspruch 8, wobei das Glühen in Schritt (e) bei einer Temperatur von mindestens 200°C für mindestens eine Stunde, vorzugsweise mindestens zwei Stunden, erfolgt.

**11.** Das Verfahren nach einem der Ansprüche 7 bis 10, wobei Schritt (c) das Hinzufügen von NaOH umfasst, um den pH-Wert auf mindestens 12 zu erhöhen.

**12.** Das Verfahren nach einem der Ansprüche 7 bis 11, wobei Schritt (d) das Hinzufügen von HCl umfasst, um den pH-Wert auf einen Wert zwischen 6 und 8 zu senken.

**Revendications**

**1.** Un matériau d'oxyde métallique comprenant :

des nanobâtonnets de ZnO (2) ; et
des particules de cuivre oxydé attachées aux nanobâtonnets de ZnO (2), les particules de cuivre oxydé (6) étant des nanoplaques s'étendant transversalement aux nanobâtonnets de ZnO ;
**caractérisé en ce que**
lesdites nanoplaques transversales de cuivre oxydé ont une normale parallèle à un axe longitudinal des nanobâtonnets de ZnO ou inclinée par rapport audit axe longitudinal de pas plus de 45° ; et **en ce que**
les nanobâtonnets de ZnO (2) pourvus de nanoplaques transversales de cuivre oxydé (4 ; 6) ont un nombre de nanoplaques transversales de cuivre oxydé compris entre 10 et 100 par $\mu$m de longueur de nanobâtonnet de ZnO.

**2.** Le matériau d'oxyde métallique selon la revendication 1, dans lequel les nanobâtonnets de ZnO (2) ont une longueur comprise entre 1 et 10 $\mu$m.

**3.** Le matériau d'oxyde métallique selon l'une des revendications 1 et 2, dans lequel les nanobâtonnets de ZnO (2) ont un diamètre compris entre 50 et 600 nm.

**4.** Le matériau d'oxyde métallique selon l'une quelconque des revendications 1 à 3, dans lequel les nanoplaques transversales de cuivre oxydé (6) sont perpendiculaires à l'axe longitudinal des nanobâtonnets de ZnO avec une tolérance de $\pm 15°$, de préférence avec une tolérance de $\pm 10°$.

**5.** Le matériau d'oxyde métallique selon l'une quelconque des revendications 1 à 4, dans lequel les nanobâtonnets de ZnO (2) pourvus de nanoplaques transversales de cuivre oxydé (4 ; 6) sont obtenables par le procédé selon l'une quelconque des revendications 7 à 12.

**6.** Un capteur de gaz (8 ; 108) comprenant : un substrat (10 ; 110) ; au moins deux électrodes (12, 16 ; 112, 116) déposées sur le substrat (10 ; 110) ; une couche de détection de gaz (20) déposée sur les au moins deux électrodes (12, 16 ; 112, 116) et sur le substrat (10 ; 110) entre lesdites au moins deux électrodes, ladite couche de détection de gaz (20) comprenant le matériau d'oxyde métallique selon l'une quelconque des revendications 1 à 5 et présentant une résistivité électrique qui varie lorsqu'elle est en contact avec le gaz étant au moins l'un de $O_2$, $H_2$, CO, éthanol et $NO_2$.

**7.** Un procédé de synthèse de nanobâtonnets de ZnO pourvus de nanoplaques à base de Cu, comprenant les étapes successives suivantes :

(a) préparer des nanobâtonnets de ZnO ;
(b) mélanger les nanobâtonnets de ZnO avec un précurseur de Cu dans une solution
(c) augmenter le pH de la solution, de manière à former des nanofils de $Cu(OH)_2$ dans la solution ;
(d) abaisser le pH de la solution, de manière à former des nanoplaques de $Cu_2(OH)_3Cl$ (4) sur les nanobâtonnets de ZnO (2).

8. Le procédé selon la revendication 7, comprenant l'étape supplémentaire suivante :
(e) recuire les nanobâtonnets de ZnO (2) pourvus des nanoplaques de $Cu_2(OH)_3Cl$ (4) de manière à transformer au moins partiellement les nanoplaques de $Cu_2(OH)_3Cl$ (4) en nanoplaques de CuO (6).

9. Le procédé selon la revendication 8, dans lequel le recuit à l'étape (e) est réalisé à une température d'au moins 100°C pendant au moins une heure, de préférence au moins deux heures.

10. Le procédé selon la revendication 8, dans lequel le recuit à l'étape (e) est réalisé à une température d'au moins 200°C pendant au moins une heure, de préférence au moins deux heures.

11. Le procédé selon l'une quelconque des revendications 7 à 10, dans lequel l'étape (c) comprend l'ajout de NaOH de manière à augmenter le pH à au moins 12.

12. Le procédé selon l'une quelconque des revendications 7 à 11, dans lequel l'étape (d) comprend l'ajout de HCl de manière à abaisser le pH à une valeur comprise entre 6 et 8.

[Fig. 1]

[Fig. 2]

[Fig. 3]

[Fig. 4]

[Fig. 5]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 106814113 A
- CN 107537501 A
- WO 2019057786 A1

### Non-patent literature cited in the description

- **RUTUJA BHUSARI** ; **JEAN-SÉBASTIEN THOMANN** ; **JÉRÔME GUILLOT** ; **RENAUD LETURCQ**. Morphology control of copper hydroxide based nanostructures in liquid phase synthesis. *Journal of Crystal Growth*, 2021, vol. 570, ISSN 0022-0248, 126225, https://doi.org/10.1016/j.icrysgro.2021.126225 (https://www.sciencedirect.com/science/article/pii/S0022024821002001
- Heteroepitaxial ZnO/CuO thin film and nanorods array: photoconductivity and field emission effect. **RAHMATI ALI et al.** JOURNAL OF MATERIALS SCIENCE: MATERIALS IN ELECTRONICS. CHAPMAN AND HALL, 26 May 2017, vol. 28, 13032-13040
- CuO nanosheets/ZnO nanorods synthesized by a template-free hydrothermal approach and their optical and magnetic characteristics. **LU PING et al.** CERAMICS INTERNATIONAL. ELSEVIER, 28 April 2017, vol. 43, 9798-9805
- **SIMON QUENTIN et al.** CuO/ZnO Nanocomposite Gas Sensors Developed by a Plasma-Assisted Route. *CHEMPHYSCHEM*, 18 June 2012, vol. 13 (9), ISSN 1439-4235, 2342-2348